# EUROPEAN PATENT APPLICATION

(11) **EP 3 006 026 A1**
(43) Date of publication of application: **13.04.2016**
(21) Application number: 15187371.8
(22) Date of filing: 29.09.2015
(51) Int. Cl.: A61K 31/198, A61K 31/7028, A61K 31/352, A61K 33/06, A61K 36/185, A61K 36/539, A61K 36/82, A23L 33/105, A61K 9/20, A61K 9/24, A61P 25/22

(54) **PHARMACEUTICAL COMPOSITION FOR TREATING ANXIETY CONTAINING L-THEANINE AND BAICALEIN**

(30) Priority: 30.09.2014 IT MI20141708
(71) Applicant: FB HEALTH S.P.A., 63100 Ascoli Piceno (IT)
(72) Inventor: SCAPAGNINI, Giovanni, 95131 CATANIA (IT); MARCHETTI, Marco, 63074 SAN BENEDETTO DEL TRONTO (IT); DE GROSSI MAZZORIN, Federico, 00123 ROMA (IT)
(74) Representative: Coppo, Alessandro

(57) **Abstract**

The present invention in one aspect concerns a composition for preventing and/or treating anxiety disorders comprising a synergistic combination of L-theanine and a flavonoid in a physiologically acceptable or edible carrier. The composition can be a nutraceutical, a herbal medicinal or a pharmaceutical product.

## Description

### FIELD OF THE INVENTION

The present invention relates to a composition for preventing or treating anxiety disorders.

The present invention originates in the field of nutraceutical, phyto-products and pharmaceutical products.

In particular, the present invention relates to a composition that suitable in the prevention or treatment of anxiety and of the associated symptoms.

### PRIOR ART

It is known that anxiety is a mental state of an individual characterised by a feeling of worry or fear that can be linked to a specific stimulus, or failure of the organism to adapt to any source of stress for this individual.

When the symptoms related to anxiety of a subject reach a threshold value beyond which they start to influence the performance of daily activities it is usually necessary to establish a suitable therapy.

The type of therapeutic treatment is generally selected as a function of the severity of the symptoms and of the individual's age.

Minor forms of anxiety are generally treated using homeopathy or herbal medicinal products that contain active ingredients of plant origin having a calming and/or anxiolytic action, such extracts of valerian, linden, hawthorn, lemon balm and chamomile.

Vice versa, pharmacotherapy is reserved for the treatment of acute or more severe forms, in which the symptoms also have a significant influence on the individual's quality of life and night rest.

Drugs commonly used for the treatment of anxiety are divided into two macrocategories: anxiolytics and antidepressants.

The most commonly used anxiolytics include benzodiazepines, molecules formed of a benzene ring fused to a diazepine ring. Benzodiazepines are classified according to the plasma half-life on which the duration of action depends. The parent of benzodiazepines is diazepam.

The use of benzodiazepines in the treatment of forms of anxiety is widespread. However, the administration of these drugs even for relatively short periods of time exposes patients to risks of dependency and addiction. Therefore, their use should be limited to short term treatment of forms of anxiety and to managing the acute phases.

However, there are currently no valid pharmacological alternatives for managing minor forms of anxiety or for establishing a long term supportive therapy, as in the majority of cases, the natural remedies currently available provide insufficient therapeutic response.

Therefore, there is currently the need for remedies based on active ingredients that are not of synthetic origin and are not based on benzodiazepine, capable of providing a suitable therapeutic response that allows management of minor forms of anxiety and of sleep disorders.

A general object of the present invention is to provide a composition suitable to improve the clinical and/or symptomatic picture related to anxiety disorders.

Another object of the present invention consists in providing a composition with an anxiolytic action based on active ingredients of natural origin that have virtually no side effects on the human organism and that can be used for the long-term treatment of anxiety and of generalised anxiety.

A further object of the present invention is represented by a treatment method for preventing or treating anxiety and/or the symptoms associated therewith.

### SUMMARY OF THE INVENTION

In accordance with a first aspect of the present invention, the inventors have surprisingly found that the combination of L-theanine and baicalein, two active ingredients of plant origin, provides a synergistic anxiolytic effect, without causing a state of sedation or a dependency typical of pharmacological treatments with benzodiazepines.

The inventors have also observed that the synergistic anxiolytic effect found is only in part due to the combined action of increase in the levels of the neurotransmitter GABA resulting from the combined action of activation of the GABA(A) receptors of the L-theanine and baicalein combination.

In accordance with a first aspect, there is thus provided a composition in accordance with the subject matter claimed in the appended claim 1.

Further embodiments of the composition and of its uses in accordance with the invention are indicated in claims 2-10 appended hereto.

In accordance with a first aspect, the present invention thus provides a composition for preventing and/or treating anxiety disorders.

In accordance with a first aspect, the present invention thus concerns a composition comprising a combination of L-theanine and a flavonoid and a physiologically acceptable carrier, said flavonoid being characterised by being baicalein optionally in glucuronide form.

In accordance with further aspects of the invention, the inventors have also found that the synergistic anxiolytic action referred to the combination of baicalein and L-theanine is further strengthened by a combination with chrysin, an additional bioflavonoid of plant origin.

In accordance with some embodiments, the composition of the invention therefore comprises baicalein and/or a glucuronide thereof, L-theanine, chrysin and a physiologically acceptable carrier.

Typically in the composition of the invention the active ingredients with anxiolytic action are provided in a pharmaceutically or nutraceutically effective amount.

The composition of the invention can be used in the prevention or in the treatment of generalised anxiety disorder and/or as adjuvant of the symptoms associated with this condition.

According to some embodiments, the composition of the invention is a pharmaceutical composition, a herbal medicinal product, a supplement or a nutraceutical product that can be added to the diet of a person suffering from anxiety disorders.

According to some aspects of the invention, there is provided a preparation or kit containing baicalein and/or a glucuronide thereof, L-theanine, optionally chrysin and a physiologically acceptable carrier as combined preparation for simultaneous, separate or subsequent use in the prevention or treatment of anxiety disorders.

In accordance with another aspect there is provided a composition comprising baicalein and/ or a glucuronide thereof, L-theanine, optionally chrysin and an edible and/or a physiologically acceptable carrier for use in the prevention or treatment of anxiety disorders and/or to induce sleep.

In accordance with a further aspect, the present invention concerns a method for preventing or treating an anxiety disorder comprising administrating to an individual a composition based on baicalein and/or a glucuronide thereof, L-theanine, optionally chrysin and an edible and/or a physiologically acceptable carrier.

The present invention will be described in detail below with reference to the accompanying figure.

### BRIEF DESCRIPTION OF THE FIGURE

Some features and advantages of the present invention will be more apparent from the accompanying figure, which represents through histograms the synergistic therapeutic response of the combination L-theanine + baicalein compared to that of the two components and to diazepam, on the murine model of anxiety indicated in example 3.

### DETAILED DESCRIPTION OF THE INVENTION

In accordance with some aspects of the invention, the inventors have discovered that the combined administration of baicalein and L-theanine produces a synergistic effect of regulation of the central receptors of benzodiazepines with a resulting powerful anxiolytic action.

The synergistic anxiolytic activity of the baicalein and L-theanine mixture has been successful proved in experiments in a preclinical murine model of anxiety. The experimentally verified anxiolytic activity was statistically significant and unexpectedly greater than the activity attributable to the sum of the activity of the two compounds taken individually.

This synergism of anxiolytic action also has the considerably advantage of producing an anxiolytic effect using a significantly lower amount than would be necessary to obtain the same therapeutic effect as the L-theanine taken individually and of minimizing any problems of dependency and addiction.

In accordance with one aspect, the present invention thus relates to a composition for use in the prevention or treatment of an anxiety disorder that contains a combination of L-theanine and a flavonoid, in particular a flavone and a physiologically acceptable carrier, said flavone being characterised by being baicalein, optionally in glucuronide form.

A bioactive ingredient or component of the composition of the invention is L-theanine.

The L-theanine is N-ethyl-L-glutamine, an amino acid correlated to glutamine that can pass through the blood-brain barrier and perform an activity at central nervous system level.

At central level it has proved to have an affinity for the AMPA, NMDA and Kainate receptors and to increase GABA production and dopamine levels. L-theanine may be obtained through extraction from plants and from fungal species, for example from the basidiomycete *Boletus badius.*

Typically, L-theanine may be obtained by extraction from the *Camellia sinensis* plant and can conveniently be obtained through hot extraction from leaves of black, green or white tea, or also from fungi.

Typically the term L-theanine is intended as L- (S-) enantiomer having pharmacological activity.

In some embodiments, the composition of the invention contains L-theanine in an amount from 10 to 1000mg, from 50 to 800, from 100 to 400mg.

In certain embodiments, the composition of the invention contains L-theanine in a quantity from 0.001 to 20% by weight, from 0.01 to 25% by weight or from 0.1 to 10% by weight.

Another component of the composition with synergistic anxiolytic action of the invention is baicalein.

Baicalein is a flavone in the class of flavonoids, identified with the IUPAC name 5,6,7-trihydroxy-2-phenyl-chromen-4-one.

Within the scope of the invention, the term baicalein designates the molecule per se and its derivatives as its glucuronide baicalin.

Typically baicalein and its derivatives are obtained as plant extracts, for example through extraction from a plant in the Lamiaceae family, typically of the *Scutellaria* genus, for example *Scutellaria lateriflora* or *Scutellaria baicalensis* or from the *Oroxylum indicum* plant.

Within the scope of the invention it is possible to use baicalein or a plant extract from a plant that contains it.

Baicalein in its different forms or derivatives increases the action of L-theanine, producing a synergistic anxiolytic effect.

According to some embodiments, the composition of the invention comprises baicalein, for example contained in a plant extract.

A suitable plant extract containing baicalein may be obtained through extraction from a part of plant belonging to the *Scutellaria* genus, in particular from the roots. In some embodiments, the plant extract is a dry extract, for example titrated to 95% in baicalein, obtained by grinding and drying a portion of the Scutellaria plant, in particular the roots.

In certain embodiments, extraction of baicalein from a portion of *Scutellaria* plant can be implemented using conventional techniques, for example through extraction with a suitable solvent from portions of the plant, for example as indicated by Bohlman and Zdero in Phytochemistry 1982; 21:2543-49.

In some embodiments, the composition of the invention contains a dry plant extract of the Scutellaria genus, titrated to 95% of Baicalein.

In certain embodiments, the composition of the invention contains baicalein in an amount from 0.1 to 1000mg, from 5 to 500, from 10 to 100mg.

In certain embodiments, the composition of the invention contains baicalin in an amount from 0.001 to 30% by weight, from 0.01 to 15% by weight or from 0.1 to 10% by weight.

The inventors have found that L-theanine and baicalein are both allosteric modulators of GABA(A), a receptor having a different interaction site than those of benzodiazepines.

There is experimental evidence that suggests that the synergistic effect of reducing anxiety obtained in individuals treated with a combination of baicalein and L-theanine could be based on this mechanism of action.

In the present application, the term "synergism or synergistic activity" is understood to mean an activity that is greater than the sum of the activities of the single active ingredient. In particular, synergism occurs when at least two substances or active ingredients interact so as to strengthen or increase one or more of their effects.

Therefore, two active substances or ingredients that produce overtly similar effects will sometimes produce exaggerated or diminished effects when used concurrently, and a quantitative assessment is necessary to distinguish these cases from a simple additive action (Tallarida RJ. Drug Synergism: its detection and applications. J PharmacolExpTher. 2001 Sep:298(3):865-72).

The inventors then found that the synergistic effect of L-theanine and baicalein is unexpectedly amplified by the presence in the formulation of chrysin an additional selected flavonoid.

According to some embodiments, the composition of the invention includes L-theanine, baicalein and chrysin and a physiologically acceptable carrier.

Chrysin is a flavonoid, typically obtained through extraction from a plant belonging to the Passiflora genus. A typical example of species from which chrysin can be extracted is Passiflora incarnata L.

A suitable plant extract containing chrysin can be obtained through extraction from a part of the Passiflora plant.

In some embodiments, the plant extract containing chrysin is a dry extract obtained through grinding and drying of a portion of the plant.

By way of example, a suitable dry extract of Passiflora may be obtained through an extraction method comprising the following steps:
- shredding/crushing of a portion of Passiflora,
- extraction with a physiologically acceptable solvent such as water or ethanol or water/alcohol mixture, for example performed from two to four times each for a period of time from 5 minutes to 2 hours, typically 30 minutes,
- filtration,
- concentration of the filtrate, for example in a vacuum with concentration eluent,
- drying, for example by means of spray or spray drying, optionally
- grinding, with optional passage of the extract obtained through a sieve or screen.

In other embodiments, the composition of the invention contains a dry extract of Passiflora, titrated to 95% of chrysin, for example in an amount from 0.1 to 1000mg, from 10 to 500, from 50 to 300mg.

In certain embodiments, the composition of the invention contains chrysin in an amount from 0.001 to 10% by weight, from 0.01 to 5% by weight or from 0.1 to 1 % by weight.

In particular, the authors observed in a murine model of anxiety that the administration of a combination of baicalein, L-theanine and chrysin exhibits an anxiolytic effect several times stronger than the sum of the three active ingredients taken individually at similar doses. Similar studies on animal models showed that the administration of two or three components of the formulation provides a therapeutic response on anxiety comparable to that of diazepam, without however causing the sedation following the administration of benzodiazepine and reducing the risk of developing dependency.

In some embodiments, the passiflora and baicalin, typically extracted from Scutellaria, present in the composition of the invention are supplied in the form of dry plant extracts.

In some embodiments, the composition of the invention comprises one or more further active substances or ingredients.

In some embodiments, the composition of the invention further comprises one or more vitamins, such as B group vitamins, niacin, vitamin A, vitamin C, vitamin PP, B group vitamins being preferred. According to some embodiments, the composition of the invention further comprises one or more micronutrients and/or minerals such as salts of Mg, K, Na, Zn, Fe, Cr, Se, Mn and others.

In particular, magnesium is an element that, within the scope of the formulation of the invention, helps to reduce or modulate anxiety. In fact, magnesium is a microelement that has a calcium-antagonist activity and contributes to stabilizing the cell membranes and promoting transmembrane ion transfer, playing an important role in the contraction and nerve conduction process. Its presence in the formulation can therefore have a relaxing action on muscle tone, which in subjects affected by anxiety can exceed physiological values.

Consequently, in certain embodiments the composition of the invention comprises magnesium or its salts with inorganic or organic acids or magnesium hydroxide. For example, the composition of the invention can comprise magnesium salts with fatty acids.

The term "carrier" as used in the present invention indicates a medium, excipient or diluent with which the association of therapeutic or active ingredients is administered.

Any carrier and/or excipient suitable for the form of preparation required for administration to human beings is contemplated for use with the compounds described in the present invention.

For the purposes of the present application, the term "physiologically acceptable" is meant to indicate edible substances that are approved by the health authorities for use in pharmaceutical, herbal medicinal, nutritional or food applications. A physiologically acceptable carrier can be a pharmaceutically acceptable carrier. Within the scope of the present application, the term combination is understood to mean that one or more active ingredients are added to or mixed with one or more other ingredients.

Therefore, the term combination or association should not be understood in the sense that the active ingredients are associated with one another with the formation of bonds of chemical or other type.

The compositions of the present invention comprise any composition produced by administering the association of active ingredients of the present invention and a physiologically or pharmaceutically acceptable carrier. These compositions are suitable for food, nutritional, pharmaceutical or dietary use in mammals, particularly in human beings.

The composition of the invention can take a wide variety of forms of preparation, according to the desired administration route.

The composition of the invention can be in solid form.

When the composition of the invention is in solid form, it can be in tablet, capsule, powder or granular form.

When the composition is in liquid form, it can be in the form of suspension, emulsion or solution. In these cases, the carrier is a liquid and can be selected, for example, from water, glycols, oils, alcohol and mixtures thereof.

Typically, the compositions of the invention are in solid form, preferably in tablet form.

The preparations in solid form may comprise one or more carriers, such as starches, sugars, microcrystalline cellulose, and optionally diluents, granulation agents, lubricants, binders or disintegrating agents.

Typically the tablets, pills, capsules or granules can also contain a binder such as tragacanth gum, acacia, corn starch or gelatine; excipients such as dicalcium phosphate; a disintegrating agent such as corn starch, potato starch, alginic acid; a lubricant such as magnesium stearate; a sweetener such as sucrose, lactose or saccharine. If desired, the tablets can be coated using conventional techniques. When the pharmaceutical unit form is a tablet, it can also contain in addition to the materials of the aforesaid type a liquid carrier such as fatty oil.

In accordance with certain embodiments, the composition of the invention contains a cellulose based excipient comprising i) organic esters of cellulose for example selected from cellulose acetate, cellulose propionate, cellulose triacetate, cellulose acetate propionate, cellulose acetate butyrate, ii) inorganic esters of cellulose, for example selected from nitrocellulose, cellulose sulphate, iii) esters of cellulose selected from a) alkyl ethers of cellulose for example selected from methyl cellulose, ethyl cellulose, ethyl methyl cellulose; b) hydroxy alkyl ethers of cellulose, for example selected from hydroxy ethyl cellulose, hydroxy propyl cellulose, hydroxy propyl cellulose, hydroxy ethyl cellulose, hydroxy propyl methyl cellulose, ethyl hydroxyethyl cellulose; c) carboxyalkyl cellulose, for example carboxymethyl cellulose, their salts and their mixtures. In certain embodiments the cellulose based excipients are cross-linked with physiologically acceptable crosslinking agents

The composition can also contain flavouring agents, preservatives, colorants and the like.

In accordance with certain embodiments, the composition is in a form for modulated or controlled release of the active ingredients. By way of example, the composition can be a delayed release, fast release or slow-fast release formulation. Typically, the formulations with modulated or controlled release contain one or more cellulose based excipients, in particular of the type described previously.

In certain embodiments, the composition of the invention comprises as excipient a hydrogenated fatty acid, preferably having a chain with from 3 to 20 carbon atoms, from 14 to 18 carbon atoms. A typical example of suitable hydrogenated fatty acid is hydrogenated palm oil.

In some embodiments, the composition is a slow-fast modulated release formulation, which preferably contains a cellulose based excipient and optionally a hydrogenated fatty acid, for example of the type described previously.

In some embodiments, the plant extracts or active ingredients contained in the composition of the present invention can be combined or mixed as active ingredients in intimate mixture with a suitable edible carrier and/or an excipient according to pharmaceutical or conventional food or nutrition industry technologies.

The compositions for pharmaceutical or nutritional use can be suitably presented in single pharmaceutical form and prepared using any one of the methods well known in the pharmaceutical or food technology.

The amount of active compound in the compositions of the invention is such as to obtain a dose that prevents or is therapeutically effective on anxiety.

In some embodiments, the composition of the invention also comprises one or more additional components such as additives, fillers, stabilizers, emulsifiers, texturing agents, film coating agents, plasticizers, wetting agents and thickeners. Various other materials can be present as coating or to modify the physical form of the pharmaceutical unit. For example, the tablets can be coated with shellac, sugar or both. To prevent disintegration during transit through the upper part of the gastrointestinal tract, the composition can be a formulation with enteric coating.

In certain embodiments in which the formulation is in liquid form, for example in cases of a syrup or elixir, in addition to the association of active ingredients, it may contain sucrose as sweetening agent, methyl and propylparabens as preservatives, a colorant and a flavouring agent such as cherry or orange flavour. In some embodiments, in the composition of the present invention, the active ingredients are usually formulated in dosage units. The dosage unit can contain from 0.1 to 1.00 mg of active ingredient or of plant extract containing the active ingredient per dosage unit for daily administration.

In some embodiments, the formulation will contain amounts of active ingredient dependent on the severity of the form of anxiety and on the related symptoms, on the condition, on other ongoing therapies, on individual state of health and on the response to the association of active ingredients.

In some embodiments, the dose is in the range from 0.001% by weight to around 60% by weight of the formulation.

According to some embodiments, the composition of the invention is a medicine. According to some embodiments, the composition of the invention is a supplement, a nutritional, nutraceutical, dietary or herbal medicinal product.

The following examples are provided mainly to illustrate the present invention and are not intended as limiting to the scope of protection as defined by the appended claims.

### EXAMPLE 1

Composition in tablet form for treating and managing anxiety

| | |
|---|---|
| L-theanine | 200 mg |
| Dry extract of Scutellaria | 50 mg equal to 47.5 mg baicalin |

The daily dose is 1 tablet per day.

### EXAMPLE 2

Composition in tablet form for treating and managing anxiety and sleep disorders

| | |
|---|---|
| L-theanine | 200 mg |
| Dry extract of Passiflora | 100 mg |
| Dry extract of Scutellaria | 50 mg of which 47.5 mg baicalin |
| Magnesium | 75 mg |

The daily dose is 1 or 2 tablets per day.

### EXAMPLE 3

Composition in modulated release tablet for treating anxiety and related symptoms

| SLOW LAYER | mg/tab. |
|---|---|
| Calcium phosphate | 130 |
| L-theanine | 100 |
| Fatty acids | 60 |
| Dry extract of Passiflora (Passiflora incarnata L., herba c. floribus) | 50 |
| Dry extract of Scutellaria (Scutellaria baicalensis Georgi, radix) titrated in baicalin | 50 |
| Microcrystalline cellulose | 30 |
| Maltodextrin | 19.65 |
| Silicon dioxide | 4.5 |
| Magnesium salts of fatty acids | 4.5 |
| Colorant: iron oxide | 1.05 |
| Colorant: E 133 | 0.3 |
| Total : | 450 |

| FAST LAYER | mg |
|---|---|
| Magnesium hydroxide | 181.6 |
| L-theanine | 100 |
| Dry extract of Passiflora (Passiflora incarnata L., herba c. floribus) | 50 |
| Maltodextrin | 26.38 |
| Microcrystalline cellulose | 20 |
| Cross-linked sodium carboxymethylcellulose | 8 |
| Colorant: iron oxide | 4.7 |
| Magnesium salts of fatty acids | 4 |
| Silicon dioxide | 4 |
| Colorant: E 133 | 1.32 |
| Total: | 400 |

| FILM COATING | mg |
|---|---|
| Ethylcellulose | 2.2 |
| Talc | 0.2 |
| Carnauba wax | 0.1 |
| Total : | 2.5 |

### EXAMPLE 4

Comparative experimental test of synergistic activity of L-theanine and baicalein. The activity of a combination of L-theanine and baicalein was tested in a murine model of anxiety.

The mice were assigned randomly to different treatment groups and subjected to tests in a variable order.

Diazepam (Hoffmann - La Roche) was used as reference product. The diazepam was diluted to a concentration of 1.5 mg/10 ml with deionised water (Millipore quality) containing propylene glycol 0.5% (Sigma-Aldrich). Two different doses (10 mg/kg and 25 mg/kg) of bot. baicalein and L-theanine were prepared dissolving the powder in 10 ml of deionised water with propylene glycol 0.5%. All the solutions were prepared the same day as the tests and administered orally (p.o.) in a volume of 10 ml/kg by body weight of the mice. The control animals received a solution of propylene glycol 0.5%.

The animals of the experiment were treated orally with a carrier, diazepam (1.5 mg/kg, *n*=8), L-theanine (10 mg/kg, 25 mg/kg, *n*=8), baicalein (10 mg/kg, 25 mg/kg, *n*=8) or L-theanine + baicalein, in a ratio 1/1 (10 mg/kg, 25 mg/kg, *n*=8), 60 minutes prior to evaluation in the maze.

Anxiolytic activity was evaluated using the elevated maze plus (EPM) test. [ ]. The maze was composed of two open arms (31 cm x 5 cm x 1 cm) and two closed arms (31 cm x 5 cm x 15 cm) that extended from a central platform (5 cm x 5 cm) raised to a height of 40 cm from the floor. On the wooden base frame, the closed arms were made of opaque grey plastic, while the open arms were made of the same material with only one slightly raised edge. The EPM was indirectly illuminated by three 40W bulbs.

The mice were placed individually at the centre of the maze facing a closed arm and the number of entries and the time spent in the closed and open arms during an observation period of 6 minutes was recorded. Entries to the arms were defined as the entry of all four paws into an arm [ ]. The time in percentage spent on the open arms (100 x open/total time) was calculated for each animal. We also used the open field test to evaluate possible sedative or stimulating activity of the animals [ ]. The open field consisted of a rectangular opaque grey plastic arena (width 30 cm, length 27 cm, height 30 cm) and was illuminated by six 40W bulbs. The floor of the arena was divided into 6 equidistant rectangles to evaluate the number of times the lines were crossed. Groups of 8 mice were treated with diazepam (1.5 mg/kg, 3 mg/kg *p.o.*), L-theanine (25 mg/kg, 50 mg/kg *n*=8), baicalein (25 mg/kg, 50 mg/kg, *n*=8) or L-theanine + baicalein, in a 1/1 ratio (25 mg/kg, 50 mg/kg, *n*=8) or a carrier (deionised water with propylene glycol at 0.5% *p.o.*). All the solutions contained propylene glycol at 0.5% as solubilising substance for diazepam. 60 minutes after oral administration, each mouse was placed at the centre of the arena and recorded for 5 minutes.

The EPM and the open field test were both video recorded using a high resolution video camera WV-CP244 (Panasonic). The videos were analysed using the TopScan Top View Animal Behaviour Analyzing System (version 1.00; Clever Sys Inc.) by an impartial person who has no knowledge of the treatment.

Statistical analysis of the data was performed using one-way analysis of variance (ANOVA) followed by the Student-Newman-Keuls multiple comparison test. In all cases the differences were considered significant if p < 0.05.

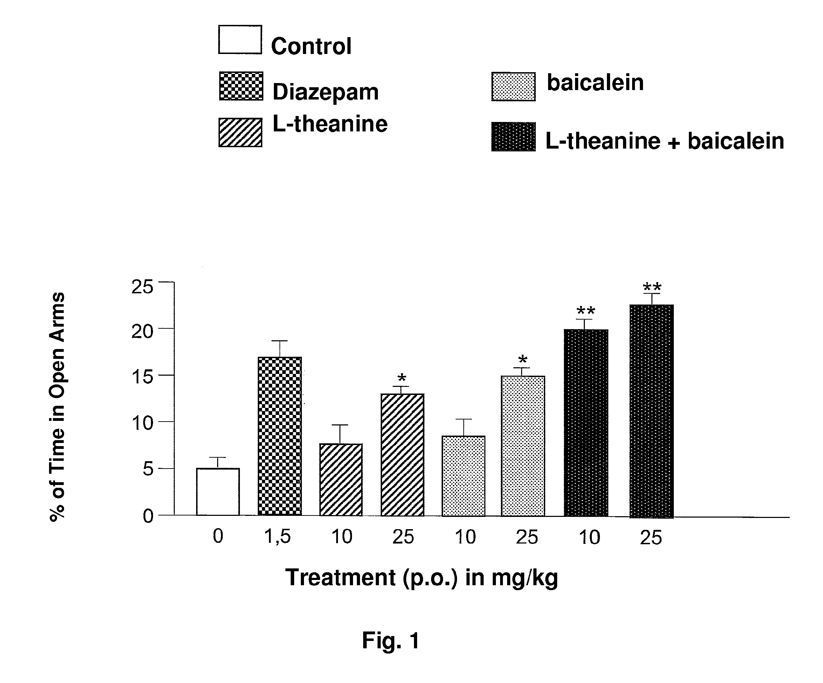

The one-way analysis of variance showed a significant increase, compared to the control, of the percentage of time spent on the closed arms, after the administration of diazepam and both L-theanine and baicalein at greater concentrations (25 mg/kg). The compounds did not show any increase in the time in percentage on the open arms at doses of 10 mg/kg. The number of entries to the open arms was greater in the group treated with diazepam. Surprisingly, the combination of the two compounds (1/1) at the total concentration of 10 mg/kg was significantly more effective compared to diazepam, and more effective than the single components at higher dosage. The combination of L-theanine and baicalein at a higher dosage (25 mg/kg in total) was slightly more effective than a lower concentration (10 mg/kg). (Fig. 1)

Diazepam at concentrations of 1.5 and 3 mg/kg caused significant sedation, as shown with the open field test (Table 1). Neither the control nor the compound used (*p.o.*) showed significant differences in relation to the number of times the lines were crossed and distance in the open field test, proving that the anxiolytic effect of the two compounds did not cause sedation.

### EXAMPLE 5

Nutraceutical product containing extracts from plants to prevent anxiety in tablet form comprising:

| | |
|---|---|
| Extract of Camellia sinensis equal to L-theanine | 50 -100 mg |
| Extract of Scutellaria baicalensis equal to baicalin | 24 - 48 mg |

## Claims

1. Composition for use in the prevention and/or in the treatment of anxiety disorder comprising a combination of L-theanine and a flavonoid as active ingredients and a physiologically acceptable carrier, said flavonoid being **characterised by** being baicalein optionally in glucuronate form.

2. Composition for use according to claim 1, wherein the baicalein in glucuronate form is baicalin.

3. Composition for use according to claim 1 or 2, wherein the baicalein and/or baicalin are contained in or derived from a plant extract of Scutellaria lateriflora.

4. Composition for use according to any one of claims 1 to 3, wherein the L-theanine is contained in or derives from a plant extract of tea, in particular green tea.

5. Composition for use according to any one of claims 1 to 4, further comprising chrysin.

6. Composition for use according to claim 5, wherein the chrysin is contained in or derives from a plant extract of Passiflora.

7. Composition for use according to any one of claims 1 to 6, further comprising magnesium.

8. Composition for use according to any one of claims 1 to 7, in the form of a food supplement, a nutraceutical product, a medicine or a herbal medicinal product.

9. Composition for use according to any one of claims 1 to 8, in the form of a tablet.

10. Composition for use according to claim 9, wherein the tablet is a tablet with modified release of the active ingredients.
